# EUROPEAN PATENT APPLICATION

(11) **EP 4 036 111 A1**
(43) Date of publication of application: **03.08.2022**
(21) Application number: 20868271.6
(22) Date of filing: 25.09.2020
(51) Int. Cl.: C07K 16/08, G01N 33/569, G01N 33/576

(54) **IMMUNOASSAY FOR HEPATITIS B VIRUS CORE-RELATED ANTIGEN AND KIT THEREFOR**

(30) Priority: 27.09.2019 JP 2019176474
(71) Applicant: Fujirebio Inc., Tokyo 163-0410 (JP)
(72) Inventor: OHUE, Chiharu, Hachioji-shi, Tokyo 192-0031 (JP); YAGI, Shintaro, Hachioji-shi, Tokyo 192-0031 (JP); AOYAGI, Katsumi, Hachioji-shi, Tokyo 192-0031 (JP)
(74) Representative: Mewburn Ellis LLP
(86) International application number: PCT/JP2020/036213
(87) International publication number: WO 2021/060450

(57) **Abstract**

Disclosed is a novel method showing a higher detection sensitivity for hepatitis B virus genotype D than those of known methods. A method of immunoassay of hepatitis B virus core-related antigen uses, as an antibody to be used for the immunoassay, a monoclonal antibody that specifically binds to at least one kind of core-related antigen of hepatitis B virus genotype D, or an antigen-binding fragment thereof, wherein an epitope of the monoclonal antibody or the antigen-binding fragment thereof is a region included in the amino acid sequence from position 31 to 48 of SEQ ID NO:3.

## Description

### TECHNICAL FIELD

The present invention relates to an immunoassay of hepatitis B virus core-related antigen (which may be hereinafter referred to as "HBcrAg"), and a kit therefor.

### BACKGROUND ART

Hepatitis B virus (which may be hereinafter referred to as "HBV") is a DNA virus having a spherical shape with a diameter of about 42 nm, and also called Dane particle. The outer side of the virus is constituted by an envelope composed of hepatitis B virus surface antigen (HBsAg), and the inner side is constituted by a core particle composed of hepatitis B virus core antigen (HBcAg) and by genes composed of incomplete circular double-stranded DNA. After the entry of HBV into a hepatocyte, the viral genes migrate into the nucleus of the hepatocyte, and the incomplete circular double-stranded DNA is converted into complete closed double-stranded DNA (covalently closed circular DNA; cccDNA). From the cccDNA, four kinds of mRNAs are transcribed, and these are translated into HBsAg, HBcAg or p22cr antigen (which may be hereinafter referred to as "p22crAg"), hepatitis B virus e antigen (which may be hereinafter referred to as "HBeAg"), and reverse transcriptase. Part of the mRNAs are incorporated as progenomic RNA into a core particle formed from HBcAg, and DNA is synthesized therein by the action of the reverse transcriptase. The core particle containing the DNA is encapsulated in the envelope formed by HBsAg, and the resulting Dane particle is released into blood. HBsAg, the empty particle, and HBeAg are released into blood in addition to the Dane particle. The empty particle herein is a particle free of DNA, and is formed by incorporation of p22crAg in the envelope. HBcrAg is a general term for HBcAg, HBeAg, and p22crAg, which are encoded together in the C gene region of HBV DNA.

Detection of HBcrAg in a test sample such as blood has been practically used as a test method for hepatitis B virus (Patent Document 1).

In the measurement of HBcrAg in a test sample such as blood, pretreatment of the test sample for reducing lowering of the value due to the influence of antibodies that are originally present in the sample and that recognize HBcrAg is also known (Patent Document 2). As a method of the pretreatment, a method in which the test sample is treated with a surfactant such as sodium dodecyl sulfate (which may be hereinafter referred to as "SDS") or an acid, and then heated, is known (Patent Document 2).

### PRIOR ART DOCUMENTS

### [Patent Documents]

[Patent Document 1] WO 2002/014871 A
[Patent Document 2] WO 2005/111620 A

### SUMMARY OF THE INVENTION

### PROBLEMS TO BE SOLVED BY THE INVENTION

In a known method that uses a monoclonal antibody described in Patent Document 1 and that has been practically used in Japan, a monoclonal antibody that specifically binds to genotype C, which is a hepatitis B virus (which may be hereinafter referred to as "HBV") genotype commonly found in Japanese, is employed. However, this method has a problem that it shows low detection sensitivity for HBV genotype D (which may be hereinafter referred to as "HBV gen.D"), which is commonly found in Europe and India.

Accordingly, an object of the present invention is to provide a novel method whose detection sensitivity for HBV gen.D is higher than those of known methods.

### MEANS FOR SOLVING THE PROBLEMS

The present inventors considered that improvement of the detection sensitivity for HBV gen.D may be possible by the use, as an antibody, of a monoclonal antibody that specifically binds to HBcrAg of HBV gen.D. However, no monoclonal antibody that specifically binds to HBcrAg of HBV gen.D has so far been prepared. The present inventors prepared a core antigen of HBV gen.D by a genetic engineering method, and mice were immunized therewith to prepare hybridomas. Monoclonal antibodies that specifically bind to HBcrAg of HBV gen.D were then successfully created therefrom. These monoclonal antibodies were used as part of antibodies for immunoassay of HBcrAg in test samples, to experimentally confirm that the detection sensitivity for HBV gen.D could be improved therewith, thereby completing the present invention.

More specifically, the present invention provides the following.
(1) A method of immunoassay of hepatitis B virus core-related antigen, the method comprising using, as an antibody to be used for the immunoassay, a monoclonal antibody that specifically binds to at least one kind of core-related antigen of hepatitis B virus genotype D, or an antigen-binding fragment thereof, wherein an epitope of the monoclonal antibody or the antigen-binding fragment thereof is a region included in the amino acid sequence from position 3 1 to 48 of SEQ ID NO:3.
(2) The method according to (1), wherein a monoclonal antibody that specifically binds to core-related antigen of hepatitis B virus genotype C, or an antigen-binding fragment thereof, is also used as an antibody to be used for the immunoassay.
(3) The method according to (1) or (2), wherein the immunoassay is a sandwich method including a first antibody and a second antibody that specifically bind to hepatitis B virus core-related antigen,
   the first antibody being a capture antibody bound to a solid phase, the second antibody being a detection antibody bound to a labeling substance,
   wherein at least one of the first antibody and the second antibody is the monoclonal antibody that specifically binds to the core-related antigen of hepatitis B virus genotype D.
(4) The method according to (3), wherein a solution containing the second antibody comprises a water-soluble polymer.
(5) The method according to any one of (1) to (4), comprising pretreating a test sample with a pretreatment liquid containing at least one selected from the group consisting of a surfactant, an acidifier, and an alkaline substance.
(6) The method according to (5), wherein the pretreatment liquid further contains a reducing agent.
(7) The method according to any one of (1) to (6), wherein the hepatitis B virus core-related antigen to be assayed by the immunoassay is a hepatitis B virus core-related antigen of genotype D.
(8) The method according to any one of (1) to (6), wherein the hepatitis B virus core-related antigen to be assayed by the immunoassay is a hepatitis B virus core-related antigen of genotype E or F.
(9) A kit for immunoassay of hepatitis B virus core-related antigen, the kit comprising, as an antibody to be used for the immunoassay, a monoclonal antibody capable of binding reaction with a core-related antigen of hepatitis B virus genotype D, or an antigen-binding fragment thereof, wherein an epitope of the monoclonal antibody or the antigen-binding fragment thereof is a region included in the amino acid sequence from position 31 to 48 of SEQ ID NO:3.
(10) The kit according to (9), further comprising a monoclonal antibody that specifically binds to a core-related antigen of hepatitis B virus genotype C, or an antigen-binding fragment thereof, as an antibody to be used for the immunoassay.

### EFFECT OF THE INVENTION

By the present invention, a novel immunoassay capable of highly sensitively detecting HBcrAg of HBV gen.D was provided for the first time.

### BRIEF DESCRIPTION OF THE DRAWINGS

Fig. 1 is a schematic diagram illustrating the relationships among the amino acid sequence structures of HBcAg, HBcAg, and p22crAg.
Fig. 2 is a diagram showing the correlation between the measurement results for the specimens in the two test examples, which measurement was carried out in the Examples of the method of the present invention.

### MODE FOR CARRYING OUT THE INVENTION

The monoclonal antibody that specifically binds to HBcrAg of HBV gen.D, used in the present invention can be prepared by the method described in detail in the Examples below. More specifically, in the Examples below, DNA was collected from blood of a patient infected with IIBV gen.D. A region encoding HBcrAg was amplified by PCR, and then introduced into *E. coli* to allow production of HBcrAg by genetic engineering. By immunizing mice with the HBcrAg, hybridomas were prepared by a conventional method. Hybridomas producing monoclonal antibodies that specifically bind to HBcrAg of HBV gen.D were selected, and the monoclonal antibodies that specifically bind to HBcrAg of HBV gen.D were collected therefrom. By this, the monoclonal antibodies that specifically bind to HBcrAg of HBV gen.D were successfully obtained. In the Examples below, two kinds of hybridomas designated HB124 and HB135 were obtained by this method. It could thus be confirmed that monoclonal antibodies that specifically bind to HBcrAg of HBV gen.D can be reproducibly prepared. The epitope of each of the monoclonal antibodies produced by HB124 and HB135 obtained in the Examples below is a region included in the amino acid sequence from position 31 to 48 of SEQ ID NO:3. A monoclonal antibody that specifically binds to a peptide including the amino acid sequence from position 31 to 48 of SEQ ID NO:3 can be judged to be a monoclonal antibody whose epitope is a region included in the amino acid sequence from position 31 to 48 of SEQ ID NO:3. In the present description and claims, "specific binding" means antigen-antibody reaction.

In the immunoassay of the present invention, a monoclonal antibody that specifically binds to HBcrAg of HBV gen.D may be used alone, but it is preferred to also use a monoclonal antibody that specifically binds to HBcrAg of another genotype. By this, HBcrAg of not only genotype D, but also the other genotype can be highly sensitively detected. For example, a method practically used in Japan employs a monoclonal antibody that specifically binds to genotype C, which is commonly found in Japanese. However, in the present invention, it is preferred to use a monoclonal antibody that specifically binds to genotype D in addition to a monoclonal antibody that specifically binds to genotype C. This allows highly sensitive detection of not only genotype C, which is commonly found in Japanese, but also genotype D, which is commonly found in Europeans and Indians.

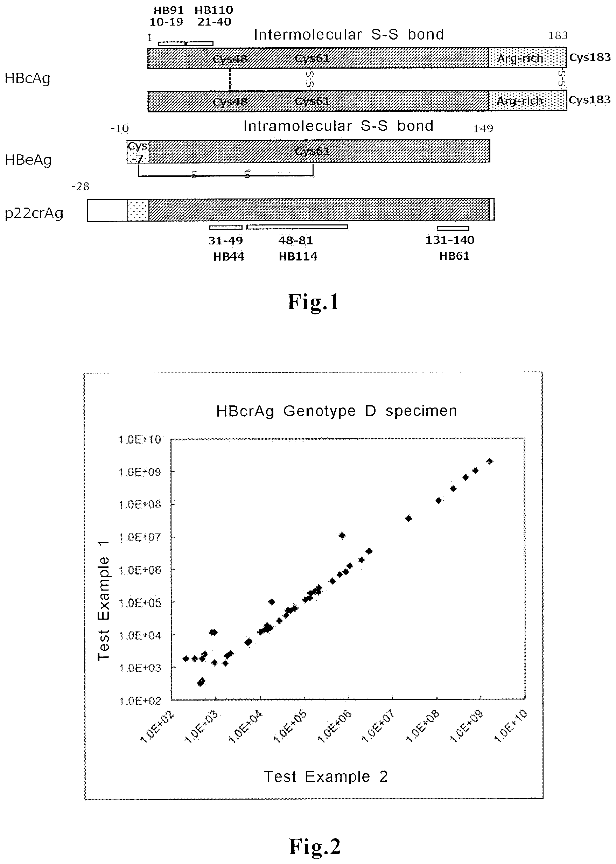

An amino acid sequence of HBcAg of HBV gen.C is shown in SEQ ID NO:1, and an amino acid sequence of HBeAg of HBV gen.C is shown in SEQ ID NO:2. The amino acids at positions 1 to 10 of SEQ ID NO:2 correspond to the amino acid sequence from position -10 to -1 of HBeAg in Fig. 1, and the amino acids at positions 11 to 159 of SEQ ID NO:2 correspond to the amino acid sequence from position 1 to 149 of HBeAg in Fig. 1. Of the amino acid sequence of HBeAg of HBV gen.D, amino acid Nos. 1 to 149 are shown in SEQ ID NO:3. Fig. 1 shows a schematic diagram illustrating the relationships among the amino acid sequence structures of HBcAg, HBeAg, and p22crAg. SEQ ID NO:4 shows the region including the epitope recognized by the monoclonal antibodies produced by the hybridomas HB124 and HB135 created in Examples. This epitope is the same between genotype C and genotype D except for the 10th amino acid (hereinafter referred to as "10aa"; the same applies hereinafter) as counted from the N-terminus of the epitope, which amino acid is Glu in genotype C, but is Asp in genotype D.

As described above, SEQ ID NO:3 is amino acid Nos. 1 to 149 of the amino acid sequence of HBcAg of HBV gen.D. The amino acid sequence from position 31 to 48 of SEQ ID NO:3 is a Minor sequence of HBV gen.D, and is also a Major sequence of HBV gen.E and HBV gen.F. Accordingly, the amino acid sequence from position 31 to 48 of SEQ ID NO:3, which is the region including the epitope of the monoclonal antibodies of the present invention, is also a sequence in the Major sequence of HBV gen.E and HBV gen.F. Thus, the monoclonal antibodies of the present invention specifically bind also to HBV gen.E and HBV gen.F. This has been experimentally confirmed in the Examples below. Thus, by the method of the present invention, genotype D, which is commonly found in HBV-infected patients in Europe and India, and genotype E and genotype F, which are commonly found in HBV-infected patients in Europe and South America, can both be highly sensitively detected.

In the immunoassay of the present invention, each monoclonal antibody described above itself may be used, or an antigen-binding fragment thereof may be used instead of, or in addition to, the monoclonal antibody. The antigen-binding fragment of the antibody is a part of the full-length antibody, and examples of the antigen-binding fragment include antibodies lacking the constant region (for example, F(ab')₂, Fab', Fab, or Fv). The antibody may also be a modified antibody such as a single-chain antibody.

The immunoassay of the present invention may be carried out in the same manner as in well-known immunoassays except that, as a monoclonal antibody that specifically binds to HBcrAg, at least the above-described monoclonal antibody that specifically binds to HBcrAg of HBV gen.D, E, or F is used. More specifically, examples of such immunoassays include the direct competitive method, indirect competitive method, and sandwich method. Other examples of such immunoassays include chemiluminescent enzyme immunoassay (CLEIA), chemiluminescence immunoassay (CLIA), turbidimetric immunoassay (TIA), enzyme immunoassay (EIA) (for example, direct competitive ELISA, indirect competitive ELISA, and sandwich ELISA), radioimmunoassay (RIA), latex agglutination, fluoroimmunoassay (FIA), and immunochromatography. These immunoassays *per se* are well known, and do not need to be described herein in detail. A brief description, however, of each immunoassay is given below.

The direct competitive method is a method in which an antibody against a target antigen to be measured (in the present invention, HBcrAg) is immobilized on a solid phase (immobilization), and blocking treatment (treatment of the solid phase with a solution of a protein such as serum albumin) for prevention of non-specific adsorption is carried out, followed by reacting this antibody with a test sample containing the target antigen and with a certain amount of labeled antigen, performing washing, and then quantifying the label bound to the solid phase. Since the antigen in the test sample and the labeled antigen competitively bind to the antibody, as the amount of the antigen in the test sample increases, the amount of the label bound to the solid phase decreases. Antigen standard solutions with various known concentrations are prepared, and the amount of the label (the absorbance, luminescence intensity, fluorescence intensity, or the like depending on the properties of the label; the same applies hereinafter) immobilized on the solid phase is measured for each solution. Thereafter, a calibration curve is prepared such that the antigen concentration is plotted along the abscissa, and the amount of the label is plotted along the ordinate. By measuring the amount of the label for an unknown test sample, and applying the measured amount of the label to the calibration curve, the amount of the antigen in the unknown test sample can be measured. The direct competitive method *per se* is well known in the art, and described in, for example, US 20150166678 A.

In the indirect competitive method, a target antigen (in the present invention, HBcrAg) is immobilized on a solid phase. Subsequently, blocking treatment of the solid phase is carried out, and then a test sample containing the target antigen is mixed with a certain amount of an anti-target-antigen antibody, followed by reacting the resulting mixture with the immobilized antigen. After washing, the anti-target-antigen antibody bound to the solid phase is quantified. This can be carried out by reacting a labeled secondary antibody against the anti-target-antigen antibody, performing washing, and then measuring the amount of the label. Antigen standard solutions with various known concentrations are prepared, and the amount of the label immobilized on the solid phase is measured for each solution. A calibration curve is then prepared. By measuring the amount of the label for an unknown test sample, and applying the measured amount of the label to the calibration curve, the amount of the antigen in the unknown test sample can be measured. It is also possible to use a labeled primary antibody without using the labeled secondary antibody. The indirect competitive method *per se* is well known in the art, and described in, for example, the above-mentioned US 20150166678 A.

The sandwich method is a method in which an anti-target-antigen antibody is immobilized on a solid phase, and blocking treatment is carried out, followed by reaction with a test sample containing a target antigen, washing, reaction with a labeled secondary antibody against the target antigen, washing, and then quantification of the label bound to the solid phase. In the sandwich method, the immobilized antibody is also referred to as "capture antibody", and the labeled antibody is also referred to as "detection antibody". Antigen standard solutions with various known concentrations are prepared, and the amount of the label immobilized on the solid phase is measured for each solution. A calibration curve is then prepared. By measuring the amount of the label for an unknown test sample, and applying the measured amount of the label to the calibration curve, the amount of the antigen in the unknown test sample can be measured. The sandwich method *per se* is well known in the art, and described in, for example, US 20150309016 A1.

Among the immunoassays described above, chemiluminescent enzyme immunoassay (CLEIA), chemiluminescence immunoassay (CLIA), enzyme immunoassay (EIA), radioimmunoassay (RIA), and fluoroimmunoassay (FIA) are immunoassays classified based on the type of the label to be used when the direct competitive method, indirect competitive method, sandwich method, or the like described above is carried out. Chemiluminescent enzyme immunoassay (CLEIA) is an immunoassay which uses an enzyme (for example, alkaline phosphatase) as the label, together with a substrate (for example, AMPPD) that generates a chemiluminescent compound. Enzyme immunoassay (EIA) is an immunoassay which uses an enzyme (for example, peroxidase, alkaline phosphatase, luciferase, or β-galactosidase) as the label. As the substrate of each enzyme, a compound quantifiable by measurement of the absorbance or the like is used. For example, in cases of peroxidase, 1,2-phenylenediamine (OPD), 3,3',5,5'-tetramethylbenzidine (TMB), or the like is used. In cases of alkaline phosphatase, *p*-nitrophenyl phosphate (pNPP) or the like is used. In cases of β-galactosidase, MG: 4-methylumbelliferyl galactoside, NG: nitrophenyl galactoside, or the like is used. In cases of luciferase, luciferin or the like is used. Radioimmunoassay (RIA) is a method which uses a radioactive substance as the label. Examples of the radioactive substance include radioactive elements such as ³H, ¹⁴C, ³²P, ³⁵S, and ¹²⁵I. Fluoroimmunoassay (FIA) is a method which uses a fluorescent substance or a fluorescent protein as the label. Examples of the fluorescent substance or the fluorescent protein include fluorescein, fluorescein isothiocyanate, rhodamine, green fluorescent protein, and red fluorescent protein. Immunoassays *per se* using these labels are well known in the art, and described in, for example, US 8039223 B and US 20150309016 A1.

Turbidimetric immunoassay (TIA) is an immunoassay which utilizes the phenomenon that an antigen-antibody complex produced by binding between a target antigen to be measured (in the present invention, HBcrAg) and an antibody against this antigen causes an increase in the turbidity. The antigen is added, at various known concentrations, to an anti-target-antigen antibody solution, and the turbidity of each resulting mixture is measured to prepare a calibration curve. By similarly measuring the turbidity of an unknown test sample, and applying the measured turbidity to the calibration curve, the amount of the antigen in the unknown test sample can be measured. Turbidimetric immunoassay *per se* is well known, and described in, for example, US 20140186238 A1. Latex agglutination method is a method similar to turbidimetric immunoassay, but uses a suspension of latex particles whose surfaces have an anti-target-antigen antibody immobilized thereon, instead of the antibody solution in turbidimetric immunoassay. Turbidimetric immunoassay and latex agglutination method *per se* are well known in the art, and described in, for example, US 7,820,398 B.

Immunochromatography is a method in which the above-described sandwich method or competitive method is carried out on a substrate (also called a matrix or a strip) formed with a porous material such as filter paper, cellulose membrane, glass fiber, or non-woven fabric. For example, in cases of immunochromatography by the sandwich method, a detection zone on which an anti-target-antigen antibody is immobilized is provided on the substrate, and a test sample containing a target antigen is added to the substrate, followed by allowing a developing liquid to flow from the upstream side. This allows the target antigen to migrate to the detection zone, and to be immobilized thereon. The immobilized target antigen is sandwiched with a labeled secondary antibody, and the label immobilized on the detection zone is detected to detect the target antigen in the test sample. By forming a label zone containing the labeled secondary antibody in the upstream side of the detection zone, the binding complex of the target antigen and the labeled secondary antibody can be immobilized on the detection zone. In cases where the label is an enzyme, a substrate zone containing a substrate of the enzyme is also provided in the upstream side of the detection zone. In cases of the competitive method, for example, the target antigen may be immobilized on the detection zone, and the target antigen in the test sample may be allowed to compete with the target antigen immobilized on the detection zone. By providing a labeled antibody zone in the upstream side of the detection zone, allowing the target antigen in the test sample to react with the labeled antibody, immobilizing unreacted labeled antibody on the detection zone, and then detecting or quantifying the label, the target antigen in the test sample can be detected or quantified. Immunochromatography *per se* is well known in the art, and described in, for example, US 6210898 B.

Among the immunoassays described above, the sandwich method is preferred from the viewpoint of the detection sensitivity and the simplicity of automation. The sandwich method is especially preferably chemiluminescent enzyme immunoassay (CLEIA), which is an immunoassay using a magnetic particle as the solid phase, an enzyme (for example, alkaline phosphatase) as the label, and a substrate (for example, 3-(2'-spiroadamantane)-4-methoxy-4-(3'-phosphoryloxy)phenyl-1,2-dioxctane disodium salt (AMPPD)) that generates a chemiluminescent compound as the substrate.

HBcAg includes an intermolecular S-S bond, and HBeAg includes an intramolecular S-S bond. They maintain particular spatial structures by these S-S bonds. For highly sensitive immunoassay of these antigens, it is preferred to cleave these S-S bonds to linearize the antigens, to dissociate the antigens from autoantibodies. Therefore, for the detection of HBcrAg, the test sample (specimen) is preferably pretreated. The following describes this pretreatment, and the reaction step and the detection step in the immunoassay after the pretreatment.

### 1. Pretreatment Step

The method of the present invention is a method in which HBcrAg present in a biological sample is measured using immunological reaction by reacting the biological sample with an antibody. The method preferably includes a pretreatment step of mixing the biological sample with a pretreatment liquid containing a surfactant, an acidifier, or an alkaline substance before the immunological reaction (reaction step). The pretreatment liquid may contain an acidifier or an alkaline substance, and a surfactant. The pretreatment step enables reduction of the lowering of the value of HBcrAg due to the influence of the antibodies that are originally present in the biological sample and that specifically bind to HBcrAg. HBcAg includes an intermolecular S-S bond, and HBeAg includes an intramolecular S-S bond. They maintain particular spatial structures by these S-S bonds. For immunoassay of these antigens, it is preferred to cleave these S-S bonds to linearize the antigens (to form linear epitopes). Thus, the pretreatment preferably includes a reducing agent for cleaving the S-S bonds. Accordingly, the pretreatment liquid preferably contains: (1) a surfactant, an acidifier, or an alkaline substance; and (2) a reducing agent.

The volume ratio between the biological sample and the pretreatment liquid to be mixed in the pretreatment step is preferably 1:10 to 10:1, more preferably 1:5 to 5:1, still more preferably 1:3 to 3:1. The biological sample to be used in the present invention is not limited as long as it is a sample that may contain HBcrAg, and examples of the biological sample include blood samples (serum, plasma, and whole blood), urine, stool, oral mucosa, pharyngeal mucosa, intestinal mucosa, and biopsy specimens (intestinal specimens and liver specimens). The biological sample is preferably a blood sample, more preferably serum or plasma.

The pretreatment liquid preferably contains a reducing agent in addition to a surfactant, an acidifier, or an alkaline substance. Preferred examples of the reducing agent contained in the pretreatment liquid include 2-diethylaminoethanethiol

(DEAET), tris(2-carboxyethyl)phosphine (TCEP), imidazole, cysteine, cysteamine, dimethylaminoethanethiol, diethylaminoethanethiol, diisopropylaminoethanethiol, and dithiothreitol. The concentration of the reducing agent, in terms of the final concentration in the mixture with the biological sample, is preferably 0.5 to 100 mM, more preferably 1.0 to 50 mM, still more preferably 2.0 to 20 mM.

When necessary, the pretreatment liquid may contain another protein denaturant such as urea or thiourea. The concentration of the denaturant, in terms of the concentration during the treatment, is preferably not less than 0.1 M, more preferably not less than 0.5 M and less than 4 M. For enhancement of the effect of the treatment, the pretreatment liquid may contain any of monosaccharides, disaccharides, citric acid, and citric acid salts, or a combination of these. The pretreatment liquid may also contain a chelating agent such as EDTA.

The pretreatment conditions can be roughly divided into the following three systems: 1. a system using a pretreatment liquid containing an acidifier as a major component (acidification pretreatment system); 2. a system using a pretreatment liquid containing a surfactant such as SDS as a major component (surfactant pretreatment system); and 3. a system using a pretreatment liquid containing an alkaline substance as a major component (alkaline pretreatment system). Any of these systems may be selected. The pretreatment systems are individually described below.

### 1-1. Acidification Pretreatment

In the acidification pretreatment system, preferred examples of the acidifier contained in the pretreatment liquid include hydrochloric acid, sulfuric acid, and acetic acid. In cases where an acidifier is used, the normality of the acid in the pretreatment liquid, in terms of the concentration during the pretreatment, is preferably not less than 0.01 N, more preferably 0.02 N to 0.5 N, still more preferably 0.05 N to 0.4 N. In cases where the normality of the acid is not less than 0.01 N, the effect of the pretreatment can be sufficiently obtained.

In the acidification pretreatment, a surfactant is preferably added in order to prevent precipitation upon the mixing with the biological sample. Examples of the type of the surfactant include cationic, zwitterionic, and nonionic surfactants.

The cationic surfactant is preferably a cationic surfactant having, in a single molecule, a single-chain alkyl group having 10 or more carbon atoms, and a tertiary amine or a quaternary ammonium salt. Examples of such a surfactant include decyltrimethylammonium chloride, dodecyltrimethylammonium chloride, tetradecyltrimethylammonium chloride, hexadecyltrimethylammonium chloride (C16TAC), decyltrimethylammonium bromide, dodecyltrimethylammonium bromide, tetradecyltrimethylammonium bromide, hexadecyltrimethylammonium bromide (C16TAB), laurylpyridinium chloride, tetradecylpyridinium chloride, and cetylpyridinium chloride. The amount of the cationic surfactant to be added, in terms of the concentration after mixing with the specimen, is preferably 0.01% to 1%, more preferably 0.01% to 0.5%.

Unless otherwise specified, each "%"-based concentration described in the present description represents a weight/volume (w/v)-based concentration.

Examples of the nonionic surfactants include polyoxyethylene alkylphenyl ether (Triton X-100 (registered trademark) or the like), Tween (registered trademark) 20, Tween 80, and polyoxyethylene alkyl ether (Brij (registered trademark) 35 or the like).

Examples of the zwitterionic surfactants include 3-[(3-cholamidopropyl)dimethylammonio]propanesulfonate (CHAPS), *N*-dodecyl-*N*,*N-*dimethyl-3-ammonio-l-propanesulfonate (C12APS), *N*-tetradecyl-*N*,*N*- dimethyl-3-ammonio-1-propanesulfonate (C14APS), and *N*-hexadecyl-*N*,*N*-dimethyl-3-ammonio-1-propanesulfonate (C16APS).

The pretreatment step may be carried out by simply mixing the biological sample with the pretreatment liquid, and leaving the resulting mixed liquid to stand at room temperature or under heat for a prescribed time. When necessary, stirring, shaking, and/or the like may be carried out. The mixed liquid is preferably heated. The heating temperature is, for example, 25°C to 45°C, preferably 30°C to 40°C. The pretreatment time is preferably not less than 1 minute, more preferably not less than 3 minutes, still more preferably not less than 5 minutes. The pretreatment time may be not more than 60 minutes although there is no upper limit of the pretreatment time.

### 1-2. Surfactant Pretreatment

In the surfactant pretreatment system, the surfactant contained in the pretreatment liquid may be at least one selected from the group consisting of nonionic surfactants, zwitterionic surfactants, anionic surfactants, and cationic surfactants. The pretreatment liquid especially preferably contains an anionic surfactant as a major component. Examples of the nonionic surfactants, zwitterionic surfactants, and cationic surfactants that may be used include those exemplified in 1-1 above. Examples of the anionic surfactants include sodium dodecyl sulfate (SDS), sodium *N*-lauroyl sarcosine (NLS), lithium dodecyl sulfate, sodium dodecylbenzcnc sulfonate (SDBS), and deoxycholic acid. In cases where SDS is used, its concentration during the pretreatment, in the mixed liquid prepared by mixing with the biological sample, is preferably 0.1 to 12.5%, more preferably 0.25 to 10%, still more preferably 0.5 to 7.5%. An SDS concentration of 0.1 to 10% is effective for sufficient release of the antigen from the antibodies in the specimen, and for suppression of the precipitation of SDS and the like.

In cases where the pretreatment liquid contains an anionic surfactant at the above-described concentration, from the viewpoint of improving the sensitivity, the pretreatment liquid preferably further contains at least one surfactant selected from the group consisting of nonionic surfactants and zwitterionic surfactants. The concentration of the nonionic surfactant, in terms of the final concentration during the pretreatment, is preferably 0.01% to 5%, more preferably 0.05% to 5%. The final concentration of the zwitterionic surfactant is preferably 0.01% to 5%.

A cationic surfactant may also be added to the mixture containing an anionic surfactant, and a nonionic surfactant or a zwitterionic surfactant. The concentration of the cationic surfactant is preferably 0.01% to 1%.

The surfactant pretreatment may be carried out by simply mixing the biological sample with the pretreatment liquid, and leaving the resulting mixed liquid to stand, or stirring or shaking the resulting mixed liquid, at room temperature or under heat. The mixed liquid is preferably heated. The heating temperature is, for example, 35°C to 95°C, preferably 60°C to 80°C. The pretreatment time may be not less than 1 minute, and may be not more than 60 minutes although there is no upper limit of the pretreatment time.

1-3. Alkaline Pretreatment

In the alkaline pretreatment system, preferred examples of the alkaline substance contained in the pretreatment liquid include: alkali metal hydroxides such as sodium hydroxide and potassium hydroxide; and alkaline earth metal hydroxides such as magnesium hydroxide. The normality of the alkaline substance in the pretreatment liquid, in terms of the final concentration during the pretreatment, is preferably more than 0.05 N and not more than 0.5 N, especially preferably 0.1 N to 0.4 N. In cases where the normality of the alkaline substance is more than 0.05 N and not more than 0.5 N, the effect of the pretreatment can be sufficiently obtained, and the influence on the subsequent reaction step can be minimized.

In the alkaline pretreatment, a surfactant may be added. Examples of the type of the surfactant include nonionic, zwitterionic, and anionic surfactants. In such a case, the sensitivity of the immunoassay described later can be further improved. Examples of the nonionic surfactants, zwitterionic surfactants, and anionic surfactants that may be used include those exemplified in 1-1 and 1-2 above.

The pretreatment step may be carried out by simply mixing the biological sample with the pretreatment liquid, and leaving the resulting mixed liquid to stand at room temperature or under heat for a prescribed time. When necessary, stirring, shaking, and/or the like may be carried out. The mixed liquid is preferably heated. The heating temperature is, for example, 25°C to 45°C, preferably 30°C to 40°C. The pretreatment time is preferably not less than 1 minute, more preferably not less than 3 minutes, still more preferably not less than 5 minutes. The pretreatment time may be not more than 60 minutes although there is no upper limit of the pretreatment time.

### 2. Reaction Step

The biological-sample mixed liquid obtained by the pretreatment step in the method of the present invention is subsequently subjected to the reaction step of immunoassay. In the reaction step, the antigen in the biological-sample mixed liquid is reacted with an antibody against HBcrAg. The biological-sample mixed liquid may be mixed with a buffer before the reaction with the antibody against HBcrAg. In cases where a pretreatment liquid containing an acidifier or an alkaline substance as a major component is used in the pretreatment step, the biological-sample mixed liquid is preferably mixed with a buffer before the reaction with the antibody against HBcrAg. For the immunoassay itself of HBcrAg, a variety of methods are well known as described above, and any immunoassay capable of quantification of HBcrAg may be employed.

Examples of the buffer include those based on MES buffer, phosphate buffer, Tris buffer, or carbonate buffer. In cases where a pretreatment liquid containing a surfactant is used, it is preferred, for the purpose of absorbing unreacted surfactant, to use a buffer containing a water-soluble polymer such as BSA, polyvinylpyrrolidone (PVP), polyvinyl alcohol (PVA), or dextran sulfate sodium at about 0.01 to 10.0%, especially 0.05 to 5.0% in terms of the final concentration after mixing with the pretreated mixed liquid. The mixed liquid of the pretreatment step and the buffer are mixed together at a volume ratio of preferably 1:10 to 10:1, more preferably 1:5 to 5:1, still more preferably 1:3 to 3:1.

The antibody against HBcrAg used in the method of the present invention is as described above. The antibody against HBcrAg may be immobilized. In the present description, an antibody that is immobilized may be simply referred to as an immobilized antibody. Examples of the solid phase include solid phases in/on which a liquid phase can be stored or loaded (for example, supports such as plates, membranes, and test tubes; and containers such as well plates, microchannels, glass capillaries, nanopillars, and monolith columns) and solid phases that can be suspended or dispersed in a liquid phase (for example, solid-phase carriers such as particles). Examples of the material of the solid phase include glasses, plastics, metals, and carbons. As the material of the solid phase, a non-magnetic material or a magnetic material may be used. From the viewpoint of the simplicity of operation and the like, the material is preferably a magnetic material. The solid phase is preferably a solid-phase carrier, more preferably a magnetic solid-phase carrier, still more preferably a magnetic particle. As the method for immobilization of the antibody, a conventionally known method may be used. Examples of such a method include physical adsorption, covalent bonding, use of an affinity substance (such as biotin or streptavidin), and ionic bonding. In a particular embodiment, the antibody against HBcrAg is an antibody immobilized on a solid phase, preferably an antibody immobilized on a magnetic solid phase, more preferably an antibody immobilized on a magnetic particle.

In the reaction step, in cases where the mixed liquid of the pretreatment step is mixed with the buffer, the resulting mixture may be brought into contact with the immobilized antibody. Alternatively, for example, an antibody immobilized on particles may be preliminarily included in a buffer to provide a particle liquid, and then the above mixed liquid may be mixed with the particle liquid. Although the reaction step may be carried out by a primary reaction step alone as in the immunoagglutination method or the competitive method, a secondary reaction step may also be provided as in the sandwich method. In cases where the secondary reaction step is provided, a washing step for removal of an unreacted component(s) may be provided between the primary reaction step and the secondary reaction step.

The antibody against HBcrAg may be labeled with a labeling substance. In the present description, an antibody labeled with a labeling substance may be simply referred to as a labeled antibody. Examples of the labeling substance include enzymes (peroxidase, alkaline phosphatase, luciferase, β-galactosidase, and the like), affinity substances (streptavidin, biotin, and the like), fluorescent substances and proteins (fluorescein, fluorescein isothiocyanate, rhodamine, green fluorescent protein, red fluorescent protein, and the like), luminescent or light-absorbing substances (luciferin, aequorin, acridinium, ruthenium, and the like), and radioactive substances (³H, ¹⁴C, ³²P, ³⁵S, ¹²⁵I, and the like). In cases where the secondary reaction is provided in the method of the present invention, the antibody to be used for the secondary reaction may be labeled with such a labeling substance.

A water-soluble polymer is preferably included in the solution (which may be hereinafter referred to as "labeled-body liquid" ) containing the antibody labeled with such a labeling substance since, in this case, the detection sensitivity can be further improved. Examples of the water-soluble polymer include dextran, aminodextran, Ficoll (trade name), dextrin, agarose, pullulan, celluloses (such as hemicellulose and lignin), chitin, chitosan, β-galactosidase, thyroglobulin, hemocyanin, polylysine, polypeptide, and DNA; and modified bodies thereof (such as DEAE Dextran and sodium dextran sulfate). Among these, Ficoll (trade name), dextran, and aminodextran, which are polysaccharides; and modified bodies thereof; are preferred. Although the weight average molecular weight of the water-soluble polymer is not limited, it is preferably 6000 to 4,000,000 from the viewpoint of the sensitivity in the immunoassay, and of the water solubility. Although the concentration of the water-soluble polymer in the labeled-body liquid is not limited, it is usually 0.5% to 10%, preferably 1% to 8% with respect to the whole labeled-body liquid from the viewpoint of the detection sensitivity.

In a particular embodiment, the method of the present invention includes, as the antibody to be used for the secondary reaction, another antibody (secondary antibody) against HBcrAg, which antibody recognizes an epitope different from that of the above-described antibody against HBcrAg. The combination of the epitope recognized by the above-described monoclonal antibody against HBcrAg and the epitope recognized by the other antibody against HBcrAg is not limited. Use of such another antibody is preferred in cases where, for example, the sandwich method is used. Although the secondary antibody may be either a polyclonal antibody or a monoclonal antibody, a monoclonal antibody is preferred from the viewpoint of the reproducibility.

### 3. Detection Step

In cases where a label is used for the primary antibody or the secondary antibody, the detection is carried out by a method suitable for the label used. For example, in cases where an enzyme label is used, the detection is carried out by adding a substrate of the enzyme. For example, in cases where alkaline phosphatase (ALP) is used for the labeled antibody, 3-(2'-spiroadamantane)-4-methoxy-4-(3'-phosphoryloxy)phenyl-1,2-dioxetane disodium salt (AMPPD) may be used as the enzyme substrate to provide a system of the chemiluminescent enzyme immunoassay (CLEIA) method.

The present invention also provides a kit for carrying out the immunoassay of the present invention described above. The kit comprises a monoclonal antibody that specifically binds to HBcrAg of HBV gen.D. The monoclonal antibody may be immobilized on a magnetic particle or the like, or may be labeled. In a preferred embodiment, the monoclonal antibody is immobilized on a magnetic particle or the like. In a preferred embodiment, the kit of the present invention may have a constitution suitable for the type of the immunoassay employed. For example, in cases where the sandwich method is employed, the kit of the present invention may include: i) the pretreatment liquid; ii) a monoclonal antibody against HBcrAg; and iii) a buffer; and, as arbitrary constituting components, iv) another antibody against HBcrAg; v) a labeling substance; vi) a diluent; and, when necessary, vii) a substrate that reacts with the labeling substance. The constituting components ii) and iii) may be contained in a single solution. The constituting component iv) may be labeled with the labeling substance v). The antibody against HBcrAg may preferably be immobilized on a magnetic particle.

The present invention is described below concretely based on Examples. However, the present invention is not limited to the following Examples.

### EXAMPLE 1

### Construction of Monoclonal Antibody That Reacts with Hepatitis B Virus Core-Related Antigen (HBcrAg) Genotype D

### (1-1) Expression and Purification of HBV Core-Related Antigen

### (A) Construction of HBc Antigen Expression Plasmid

An expression plasmid corresponding to the core region of IIBV genotype D (HBV gen.D) was constructed by the following method. After mixing 100 µL of serum of an HBV gen.D patient with 100 µL of a DNA extraction liquid [10 µL of 1M Tris-HCl (pH 8.4), 8 µL of 250 mM EDTA, 40 µL of 10% SDS, 8 µL of 5M NaCl, 10 µL of 20 mg/mL Proteinase K, 1 µL of tRNA (5 µg/µL), 23 µL of sterile water], the resulting mixture was incubated at 54°C for 30 minutes. After adding 200 µL of phenol-chloroform (1:1) solution thereto, the resulting mixture was mixed, and then centrifuged at 15,000 rpm for 5 minutes. Thereafter, the supernatant was removed therefrom, and 150 µL of isopropanol and 7 µL of 5 M NaCl were added to the supernatant, followed by leaving the resulting mixture to stand at -20°C for 1 hour. After carrying out centrifugation at 15,000 rpm at 4°C for 5 minutes, the resulting precipitate was rinsed with 70% ethanol, and then centrifuged again at 15,000 rpm at 4°C for 5 minutes. The precipitate was air-dried, and then dissolved in 20 µL of sterile water, to provide an HBV DNA solution.

Using 5 µL of the HBV DNA solution, PCR was carried out with two primers (5'-gaattcatggacattgacccgtataaa-3' (SEQ ID NO:5) and 5'-ggatcctaacattgagattcccgaga-3' (SEQ ID NO:6)). The PCR was carried out using a kit of GeneAmpTM (DNA Amplification Reagent Kit, manufactured by Perkin Elmer Cetus) under the following conditions: DNA denaturation at 95°C for 1 minute, annealing at 55°C for 1 minute, and DNA synthesis at 72°C for 1 minute. The resulting DNA fragment was separated by 0.8% agarose gel electrophoresis, and then purified by the glass powder method (GeneClean). The gene fragment amplified by this PCR encodes a region of the core of HBcrAg. Digestion of 0.5 µg of the amplified HBc gene fragment was carried out in 20 µL of a restriction enzyme reaction liquid [50 mM Tris-HCl (pH 7.5), 10 mM MgCl₂, 1 mM dithiothreitol, 100 mM NaCl, 15 units of EcoRI, and 15 units of BamHI enzyme] at 37°C for 1 hour, and then 0.8% agarose gel electrophoresis was carried out to purify an EcoRI-BamHI fragment of about 570 bp. Subsequently, 0.5 µg of DNA of the expression vector pATrp was digested in 20 µL of a restriction enzyme reaction liquid [50 mM Tris-HCl (pH 7.5), 10 mM MgCl₂, 1 mM dithiothreitol, 100 mM NaCl, 15 units of EcoRI, and 15 units of BamHI enzyme] at 37°C for 1 hour, and then 39 µL of water was added to the reaction liquid. Heat treatment was then carried out at 70°C for 5 minutes, and 1 µL of bacterial alkaline phosphatase (BAP) (250 units/µL) was added thereto, followed by incubation at 37°C for 1 hour.

Phenol was added to the resulting reaction liquid, and phenol extraction was carried out. The resulting aqueous layer was subjected to ethanol precipitation, and then the resulting precipitate was dried. The thus obtained EcoRI-BamHI-treated vector DNA, in an amount of 0.5 µg, and the above-described HBc fragment of 570 bp were mixed with 5 µL of 10× ligase buffer [660 mM Tris-HCl (pH 7.5), 66 mM MgCl₂, 100 mM dithiothreitol, and 1 mM ATP] and 1 µL of T4 ligase (350 units/µL), and then water was added thereto to a final volume of 50 µL, followed by incubation at 16°C overnight to perform ligation reaction. In order to obtain the expression plasmid pATrp-HBc, the ligation reaction liquid was used to transform E. *coli* HB101.

The competent *E. coli* strain used for the transformation is prepared by the calcium chloride method [Mandel, M. and Higa, A., J. Mol. Biol., 53, 159-162 (1970)]. The transformed *E. coli* was applied to an LB plate (1% tryptone, 0.5% NaCl, 1.5% agar) supplemented with 25 µg/mL ampicillin, and then incubated at 37°C overnight. A loopful of colonies generated on the plate were scraped, and transferred to LB medium supplemented with 25 µg/mL ampicillin. Culture was carried out at 37°C overnight.

By centrifugation of 1.5 mL of the bacterial culture, bacterial cells were collected, and DNA was extracted therefrom by the minipreparation alkaline method for plasmid DNA [Manniatis et al., Molecular Cloning: A Laboratory Manual (1982)]. Digestion of 1 µL of the obtained plasmid DNA was carried out in 20 µL of a restriction enzyme reaction liquid [50 mM Tris-HCl (pH 7.5), 10 mM MgCl₂, 1 mM dithiothreitol, 100 mM NaCl, 15 units of EcoRI, and 15 units of BamHI enzyme] at 37°C for 1 hour, and then agarose gel electrophoresis was carried out to select a pATrp-HBc expression plasmid that generates an EcoRI-BamHI fragment of about 570 bp.

### (B) Expression and Purification of Polypeptide Encoding HBc gen.D Antigen

An *E. coli* HB101 strain having the expression plasmid pATrp-HBc was inoculated to 3 mL of 2YT medium (1.6% tryptone, 1% yeast extract, 0.5% NaCl) supplemented with 50 µg/mL ampicillin, and then cultured at 37°C for 9 hours. One milliliter of the culture liquid was inoculated to 100 mL of M9-CA medium (0.6% Na₂HPO₄, 0.5% KH₂PO₄, 0.5% NaCl, 0.1% NH₄Cl, 0.1 mM CaCl₂, 2 mM MgSO₄, 0.5% casamino acid, 0.2% glucose) supplemented with 50 µg/mL ampicillin, and cultured at 37°C. When the OD600 reached 0.3, indoleacrylic acid was added thereto to a final concentration of 40 mg/L, and the culture was further carried out for 16 hours. The culture liquid was centrifuged at 5000 rpm for 10 minutes to collect bacterial cells.

To the bacterial cells, 20 mL of buffer A [50 mM Tris-HCl (pH 8.0), 1 mM EDTA, and 30 mM NaCl] was added. The cells were then suspended, and centrifuged again to obtain 2.6 g of expression bacterial cells. The obtained bacterial cells were suspended in 10 mL of buffer A, and the *E. coli* membrane was disrupted by sonication, followed by carrying out centrifugation at 12,000 rpm at 4°C for 30 minutes, to obtain a soluble fraction containing HBc particles. The collected supernatant was centrifuged (Beckman SW28.1 rotor) at 23,000 rpm at 4°C for 2 hours, to obtain a precipitate. The precipitate was resuspended in Tris-EDTA buffer (50 mM Tris-HCl (pH 8.0), 5 mM EDTA) supplemented with 5% sucrose. The resulting suspension was applied to a Sepharose CL4B (Amersham-Pharmacia Biochem) column (2.6 cm × 85 cm) equilibrated with Tris-EDTA buffer supplemented with 5% sucrose, and eluted using the same buffer. Fractions was analyzed by SDS-PAGE, followed by collection of a fraction for which a band of HBc antigen having a molecular weight of 22 kDa was detected. The collected fraction was concentrated by ultrafiltration (molecular weight cutoff, 50 kDa), and the resulting concentrate was layered on a step density gradient prepared by layering of Tris-EDTA buffers containing 60% sucrose, 50% sucrose, or 40% sucrose. Centrifugation (Beckman Ty60Ti rotor) was then carried out at 39,000 rpm at 4°C for 5 hours. Thereafter, fractions were sequentially collected from the bottom, and then analyzed by SDS-PAGE. HBc antigen was fractionated into two layers: a high-density fraction and a low-density fraction. Each fraction was collected, and used as a purified HBc antigen product.

### (1-2) Preparation of Hybridomas

SDS was added to the polypeptide (genotype D HBc) prepared by the above method, to a final concentration of 10%. Denaturation treatment was then carried out at 100°C for 5 minutes. The denatured HBc antigen was diluted to a final concentration of 0.2 to 1.0 mg/mL in 10 mM phosphate buffer (pH 7.3) supplemented with 0.15 M NaCl (PBS), and the resulting dilution was mixed with the same volume of Freund's adjuvant, followed by intraperitoneal administration of 10 to 20 µg of the resulting mixture to 4- to 6-week old BALB/c mice. A total of five times of booster immunizations were carried out at 2- to 4-week intervals, and then final immunization was carried out by administration of a solution of 10 µg of HBc in PBS to the tail vein.

On Day 3 after the final immunization, the spleen was aseptically removed from each mouse, and loosened into individual cells using scissors and a metal mesh, followed by three times of washing in RPMI-1640 medium. Cells of the mouse myeloma cell line Sp2/OAg14 at the logarithmic growth phase were washed three times with RPMI-1640 medium, and mixed with the spleen cells at a cell number ratio of 1:5. After carrying out centrifugation at 200×g for 5 minutes, the supernatant was removed. While the cell cluster was gently mixed, 1 mL of RPMI-1640 medium supplemented with 50% polyethylene glycol (PEG) 4000 (Merck) was slowly added thereto, and then 10 mL of RPMI-1640 medium was further added thereto to allow cell fusion.

The fused cells were subjected to centrifugation (200×g, 5 minutes) to remove the PEG. The cells were then suspended in RPMI-1640 medium supplemented with 10% fetal bovine serum, and with hypoxanthine, aminopterin, and thymidine (HAT), followed by plating on a 96-well cell culture plate. By about 10 days of culture, only hybridomas were grown, and then part of the culture supernatant was taken. Thereafter, the ELISA method was carried out using, as an immobilized antigen, HBc preliminarily denatured with SDS, in order to screen for wells in which anti-HBc antibody was produced. As a result, hybridomas producing monoclonal antibodies reactive with the denatured HBc were obtained. Further, the same screening was carried out in the presence of SDS, to select hybridomas producing monoclonal antibodies reactive with the denatured HBc also in the presence of SDS.

The obtained hybridomas were subjected to the limiting dilution method to obtain single clones, to establish antibody-producing hybridomas. The obtained hybridomas were designated HB124 and HB135.

### (1-3) Preparation and Analysis of Monoclonal Antibodies

Each hybridoma obtained by the method described in (1-2) was implanted in the abdominal cavity of a BALB/c mouse to which pristane had been intraperitoneally administered in advance. Seven to fourteen days later, ascites containing the produced monoclonal antibody was collected. The monoclonal antibody was subjected to affinity chromatography using a protein A-Sepharosc column, to separate and purify the IgG fraction.

Using an isotype typing kit (Zymed) that uses anti-mouse Ig isotype antibodies, the (sub)class of each monoclonal antibody was identified. As a result, HB124 was found to be IgG2b, κ; and HB135 was found to be IgG2a, κ.

Peptides each having a sequence of 20 amino acids in the amino acid sequence of the HBc antigen shown in SEQ ID NO:1 or SEQ ID NO:3 were prepared, and immobilized on a microtiter plate. The reactivity of each obtained monoclonal antibody to each peptide was investigated to perform epitope analysis.

The results are shown in Table 1. Table 1 shows the epitopes of HB44, HB50, HB61, HB91, and HB110, which are monoclonal antibodies against HBc gen.C. The preparation method for HB44, HB50, HB61, HB91, and HB110, and the epitope analysis method were as described in Patent Document 1.

Table 1 also shows the epitope analysis results for HB124 and HB135. HB124 and HB135 specifically bound to the peptide composed of positions 31 to 48 of SEQ ID NO:3. Thus, HB124 and HB135 were found to recognize a region included in the amino acid sequence from position 31 to 48 of SEQ ID NO:3 as an epitope. Since the amino acid sequence from position 31 to 48 of SEQ ID NO:3 is a sequence commonly found among HBc gen.D, HBc gen.E, and HBc gen.F, HB124 and HB135 were found to be antibodies that specifically bind to HBc gen.D, HBc gen.E, and HBc gen.F.

**[Table 1]**

| Clone name | Subclass | Assumed recognition site (amino acid positions) | SEQ ID NO (genotype) |
|---|---|---|---|
| HB44 | IgG1 | 31-49 | 1 (C) |
| HB50 | IgG1 | 168-176 | 1 (C) |
| HB61 | IgG1 | 131-140 | 1 (C) |
| HB91 | IgG1 | 1-19 | 1 (C) |
| HB110 | IgG1 | 21-40 | 1 (C) |
| HB124 | IgG2b | 31-48 | 2 (D) |
| HB135 | IgG2a | 31-48 | 2 (D) |

### EXAMPLE 2

### Measurement of HBcrAg Genotype D by Immunoassay

### (1) Preparation of Anti-HBcrAg Plates

To polystyrene 96-well microwell plates (manufactured by Nunc), 100 (µL/well of antibody dilutions (0.1 M sodium hydrogen carbonate, 0.1 M sodium chloride; pH 9.6) containing each of the anti-HBcrAg antibodies shown in Table 2 at each concentration were dispensed, and then the plates were incubated at 4°C overnight. The microwell plates were washed with PBS three times, and then 200 µL/well of a blocking solution (PBS supplemented with 1.0% BSA and 3% sucrose) was dispensed thereto, followed by incubation at room temperature for 2 hours. After removing the blocking solution, the plates were dried under vacuum, to provide anti-HBcrAg antibody plates.

**[Table 2]**

| | Epitope | Test Example 1 (µg/mL) | Test Example 2 (µg/mL) |
|---|---|---|---|
| HB44 | 31-49 (SEQ ID NO:1) | 2 | 2 |
| HB61 | 131-140 (SEQ ID NO:1) | 1 | 1 |
| HB114 | 1-81 (SEQ ID NO:1) | 1 | 1 |
| HB124 | 31-48 (SEQ ID NO:3) | 2 | 0 |

### (2) Measurement of HBcrAg Genotype D

Forty-four serum specimens positive for HBcrAg genotype D that were purchased (obtained from ProMedDx) were subjected to measurement of HBcrAg using the two kinds of plates prepared in (1). After mixing 100 µL of each specimen with 50 µL of an SDS solution (15% SDS, 2% Tween 60 (trade name)), the reaction was allowed to proceed at 70°C for 30 minutes.

To each microwell plate, 100 µL of a primary reaction buffer (100 mM Tris, 20 mM EDTA·2Na, 200 mM NaCl, 5% BSA, 1% Triton X405; pH7.5) was dispensed, and then 50 µL of each reacted specimen described above was added thereto. The resulting mixture was shaken at room temperature for 120 minutes, and then washed five times with 0.5% Tween (trade name)/PBS. Subsequently, 100 µL/well of a solution of each alkaline phosphatase-labeled anti-HBcrAg monoclonal antibody shown in Table 3 (20 mM Tris, 150 mM NaCl, 0.1% Casein Na, 1% BSA, 5.7 mM MEGA10, 3.4 mM NLS; pH7.5) was dispensed thereto, and left to stand at room temperature for 60 minutes. The alkaline phosphatase labeling of the HB91 and HB110 used herein was carried out according to a conventional method. The plate was washed five times with 0.5% Tween (trade name)/PBS, and then 100 µL/well of a substrate solution (CDP-Star (registered trademark) + Emerald II (registered trademark)) was dispensed thereto, followed by allowing the reaction to proceed at room temperature for 20 minutes, and then carrying out photometry using a microplate reader.

In addition to the above specimens, standard solutions containing known concentrations of recombinant HBcrAg were subjected to the measurement, to prepare a standard curve. Based on the luminescence signal from each specimen, the HBcrAg concentration was calculated. Fig. 2 shows the correlation between the measured values (U/mL) for each specimen under the conditions of Test Examples 1 and 2. In Fig. 2, the ordinate represents Test Example 1, and the abscissa represents Test Example 2. Of the 44 specimens that were positive for genotype D of HBcrAg, 9 specimens exhibited significant increases in the measured value under the conditions of the Example. It was shown that, by using the anti-HBcrAg monoclonal antibody HB124, the measurement sensitivity for genotype D can be increased.

In addition, a plate on which HB135 was immobilized instead of HB124 was used to measure HBcrAg of each specimen under the same conditions. As a result, the measured values of HBcrAg were highly correlated with those in the cases where HB124 was used, and almost the same results were obtained (data not shown).

**[Table 3]**

| | Epitope | Test Example 1 and Test Example 2 (both) (µg/mL) |
|---|---|---|
| HB91 | 1-19 | 0.1 |
| HB110 | 21-40 | 0.5 |

### EXAMPLE 3

### Influence of Addition of Reducing Agent to Specimen Pretreatment Liquid (Acid Treatment)

From two kinds of purchased HBcrAg-positive specimens with known concentrations of antigen (Specimen A (p22crAg-dominant specimen)) and Specimen B (HBeAg-dominant specimen)), 10⁵- and 10⁶-fold diluted specimens were provided, and these were subjected to specimen pretreatment and HBcrAg detection.

To 30 µL of each specimen, 90 µL of each pretreatment liquid shown in Table 4 was added, and the reaction was allowed to proceed at 37°C for 6.5 minutes. After adding 30 µL of a neutralization solution (0.7 M Bicine, 10% NLS; pH 10) thereto, the reaction was allowed to proceed at 37°C for 20 seconds.

Magnetic particles (manufactured by Fujirebio Inc.) on which HB44, HB124, HB61, and HB114 were immobilized at 4:1:4:7 according to a conventional method were used. To 50 µL of a particle dilution (50 mM MOPS, 8% BSA; pH 7.5) containing 0.06% antibody-immobilized magnetic particles, the treated specimen was added, and the reaction was allowed to proceed at 37°C for 8 minutes. After washing with 0.05% Tween 20 (trade name)/PBS, 50 µL of a labeled-body liquid (20 mM Tris-HCl, 300 mM NaCl, 3% BSA, 13.6 mM NLS, 5.5 mM C14APS; pH 7.5) containing 1 ug/mL of the alkaline phosphatase-labeled Fab fragment of antibody HB91 was added, and the reaction was allowed to proceed at 37°C for 8 minutes. After washing with 0.05% Tween 20 (trade name)/PBS, an AMPPD substrate solution was added, followed by measurement of the luminescence at 463 nm.

The measurement result under each condition is shown in Table 4. While the negative specimens were not influenced by the addition of the reducing agent in the pretreatment, the HBcrAg-positive specimens, including both the p22crAg-dominant specimen and the HBeAg-dominant specimen, exhibited increases in the signal. It was shown, in particular, that the antigen can be more securely detected in both specimens even at a very low antigen concentration of 2.46 to 2.74 LogU/mL ("LU/mL" in the table).

**[Table 4]**

| | Test Example | 3 | 4 | 5 | 6 | 7 | 8 | 9 | 10 | 11 | 12 | 13 | 14 | 15 |
|---|---|---|---|---|---|---|---|---|---|---|---|---|---|---|
| Pretreatment liquid composition Urea | M | 0. 711 | 0. 711 | 0. 711 | 0. 711 | 0. 711 | 0. 711 | 0. 711 | 0. 711 | 0. 711 | 0. 711 | 0. 711 | 0. 711 | 0. 711 |
| HCl | M | 0. 223 | 0. 223 | 0. 223 | 0.223 | 0. 223 | 0. 223 | 0. 223 | 0. 223 | 0. 223 | 0. 223 | 0.223 | 0. 223 | 0. 223 |
| Triton X-100 | % | 0.214 | 0. 214 | 0. 214 | 0. 214 | 0. 214 | 0. 214 | 0. 214 | 0. 214 | 0. 214 | 0. 214 | 0.214 | 0. 214 | 0. 214 |
| C16APS | mM | 3. 65 | 3. 65 | 3. 65 | 3. 65 | 3. 65 | 3. 65 | 3. 65 | 3.65 | 3. 65 | 3. 65 | 3. 65 | 3. 65 | 3. 65 |
| DEAET | mM | - | 5 | 10 | 15 | 20 | 30 | 40 | | | | | | |
| TCEP | mM | - | - | - | - | - | - | - | 5 | 10 | 15 | 20 | 30 | 40 |
| Negative specimen 1 | | 1196 | 1185 | 1195 | 1178 | 1183 | 1112 | 1165 | 1227 | 1247 | 1252 | 1294 | 1252 | 1295 |
| Negative specimen 2 | | 1156 | 1109 | 1145 | 1141 | 1176 | 1219 | 1223 | 1299 | 1297 | 1154 | 1281 | 1249 | 1238 |
| Negative specimen 3 | | 1206 | 1120 | 1197 | 1196 | 1216 | 1164 | 1217 | 1345 | 1281 | 1284 | 1333 | 1216 | 1242 |
| Specimen A × 10⁵ (3. 46 LU/mL) | | 5212 | 9366 | 10463 | 11489 | 11364 | 11908 | 11857 | 8896 | 10219 | 10945 | 11115 | 11400 | 11837 |
| Specimen A × 10⁶ (2. 46 LU/mL) | | 1626 | 2057 | 2129 | 2071 | 2118 | 2275 | 2252 | 2097 | 2111 | 2126 | 2203 | 2310 | 2321 |
| Specimen B × 10⁵ (3. 74 LU/mL) | | 8403 | 12541 | 13214 | 13556 | 13257 | 12522 | 12666 | 11406 | 12043 | 12458 | 12860 | 12838 | 12534 |
| Specimen B × 10⁵ (2. 74 LU/mL) | | 1974 | 2190 | 2286 | 2215 | 2369 | 2330 | 2338 | 2181 | 2362 | 2367 | 2362 | 2443 | 2340 |

### EXAMPLE 4

### Influence of Addition of Reducing Agent to Specimen Pretreatment (SDS Treatment)

The same sera as in Example 3 were diluted 100-fold to provide specimens, and the specimens were subjected to HBcrAg detection. To 100 µL of each specimen, 200 µL of each pretreatment liquid shown in Table 5 was added, and the reaction was allowed to proceed at 80°C for 5 minutes. Magnetic particles (manufactured by Fujirebio Inc.) on which HB44, HB124, HB61, and HB114 were immobilized at 4:1:4:7 according to a conventional method were used. To 50 µL of a particle dilution (50 mM MOPS, 8% BSA; pH 7.5) containing 0.06% antibody-immobilized magnetic particles, 50 µL of the treated specimen was added, and the reaction was allowed to proceed at 37°C for 8 minutes. After washing with 0.05% Tween 20 (trade name)/PBS, 50 µL of a labeled-body liquid (20 mM Tris-HCl, 37.5 mM NaCl, 1% BSA, 11.4 mM MEGA10, 5.1 mM NLS, 2.9 mM SDBS; pH 7.5) containing 1 µg/mL of the alkaline phosphatase-labeled Fab fragment of antibody HB91 was added, and the reaction was allowed to proceed at 37°C for 8 minutes. After washing with 0.05% Tween 20 (trade name)/PBS, an AMPPD substrate solution was added, followed by measurement of the luminescence at 463 nm.

The measurement result under each condition is shown in Table 5. The HBcrAg-positive specimens, including both the p22crAg-dominant specimen and the HBeAg-dominant specimen, exhibited increases in the signal.

**[Table 5]**

| | Test Example | 16 | 17 | 18 | 19 | 20 |
|---|---|---|---|---|---|---|
| Pretreatment liquid composition SDS | % | 10 | 10 | 10 | 10 | 10 |
| Triton X-100 | % | 0.225 | 0.225 | 0.225 | 0.225 | 0.225 |
| C14APS | % | 1.5 | 1.5 | 1.5 | 1.5 | 1.5 |
| EDTA·2Na | mM | 3.75 | 3.75 | 3.75 | 3.75 | 3.75 |
| TCEP | mM | - | 5 | 10 | 15 | 20 |
| Specimen A × 100 | | 439822 | 801827 | 827779 | 882482 | 827936 |
| Specimen B × 100 | | 865597 | 1655247 | 2095783 | 1845019 | 1448086 |

### EXAMPLE 5

### Influence of Addition of Water-Soluble Polymer to Labeled-Body Liquid

The same specimens as in Example 3 were subjected to HBcrAg detection. To 30 µL of each specimen, 90 µL of a pretreatment liquid (containing 16 mM DEAET) was added, and the reaction was allowed to proceed at 37°C for 6.5 minutes. Subsequently, 30 µL of a neutralization solution was added thereto, and the reaction was allowed to proceed at 37°C for 20 seconds.

The treated specimen was added to 50 µL of a particle dilution containing 0.06% antibody-immobilized magnetic particles prepared in the same manner as in Example 3, and the reaction was allowed to proceed at 37°C for 8 minutes. After washing with 0.05% Tween 20 (trade name)/PBS, 50 µL of each labeled-body liquid shown in Table 6 containing 1 µg/mL of the alkaline phosphatase-labeled Fab fragment of antibody HB91 was added, and the reaction was allowed to proceed at 37°C for 8 minutes. After washing with 0.05% Tween 20 (trade name)/PBS, an AMPPD substrate solution was added, followed by measurement of the luminescence at 463 nm.

The measurement result under each condition is shown in Table 6. By the addition of Ficoll to the labeled-body liquid, the signal increased in a concentration-dependent manner. The negative specimens also tended to exhibit an increased signal, giving a high background. However, the increase in the signal was more remarkable in the positive specimens. It was thus shown that the antigen can be more securely detected even at a very low concentration of 2.46 to 2.74 LU/mL.

**[Table 6]**

| | Test Example | 21 | 22 | 23 | 24 | 25 | 26 | 27 | 28 | 29 |
|---|---|---|---|---|---|---|---|---|---|---|
| Labeled-body liquid | mM | 20 | 20 | 20 | 20 | 20 | 20 | 20 | 20 | 20 |
| Tris-HCl | | | | | | | | | | |
| NaCl | mM | 300 | 300 | 300 | 300 | 300 | 300 | 300 | 300 | 300 |
| BSA | % | 3 | 3 | 3 | 3 | 3 | 3 | 3 | 3 | 3 |
| Ficoll 400 | % | - | 1 | 2 | 3 | 4 | 5 | 6 | 8 | 10 |
| NLS | % | 0.4 | 0.4 | 0. 4 | 0.4 | 0. 4 | 0. 4 | 0.4 | 0. 4 | 0. 4 |
| C14APS | % | 0.2 | 0. 2 | 0. 2 | 0.2 | 0. 2 | 0. 2 | 0. 2 | 0. 2 | 0. 2 |
| Negative specimen 1 | | 1215 | 1233 | 1193 | 1291 | 1304 | 1482 | 1472 | 1633 | 1719 |
| Negative specimen 2 | | 1161 | 1189 | 1282 | 1321 | 1363 | 1395 | 1482 | 1518 | 1720 |
| Negative specimen 3 | | 1264 | 1259 | 1179 | 1269 | 1437 | 1437 | 1564 | 1633 | 1764 |
| Specimen A × 10⁵ (3. 46 LU/mL) | | 5580 | 6036 | 6657 | 6967 | 7422 | 7831 | 8493 | 9291 | 9542 |
| Specimen A × 10⁶ (2. 46 LU/mL) | | 2660 | 2832 | 3149 | 3302 | 3527 | 3680 | 3952 | 4205 | 4255 |
| Specimen B × 10⁵ (3. 74 LU/mL) | | 3979 | 4170 | 4688 | 4819 | 5273 | 5333 | 5700 | 6056 | 6424 |
| Specimen B × 10⁶ (2. 74 LU/mL) | | 2007 | 2176 | 2312 | 2447 | 2605 | 2861 | 2808 | 3070 | 3378 |

### Measurement of HBcrAg Genotypes D, E, and F

### (1) Preparation of Anti-HBcrAg Particles

Magnetic particles (manufactured by Fujirebio Inc.) on which the antibodies HB44, HB135, HB61, and HB114 were immobilized were prepared according to the method described in Example 3. As a control, magnetic particles (manufactured by Fujirebio Inc.) on which the antibodies HB44, HB61, and HB114 were immobilized were prepared.

### (2) Measurement of Specimens of HBcrAg Genotypes D, E, and F, and Measurement of Recombinant HBeAg Genotype D

Specimens positive for HBV genotypes A, C, and D were obtained from ProMedDx. Specimens positive for genotypes E and F were obtained from TRINA. As a recombinant HBcrAg, a recombinant antigen solution containing 1 ng/mL of a recombinant antigen composed of the amino acid sequence from 1 to 149 of HBeAg genotype D was prepared. Each specimen was treated under the conditions of Test Example 5 in Example 3, to obtain a treated specimen. The recombinant antigen solution was similarly pretreated to obtain a treated antigen solution.

HBcrAg was measured by the same method as described in Example 3 except that the magnetic particles on which HB44, HB135, HB61, and HB114 were immobilized (Test Example 32) or the magnetic particles on which HB44, HB61, and HB114 were immobilized (Test Example 30), prepared in Example 6(1); or the magnetic particles on which HB44, HB124, HB61, and HB114 were immobilized (Test Example 31), prepared in Example 3; were used with the above-described treated specimens or antigen solution. Further, instead of the above specimens, standard solutions containing known concentrations of recombinant HBcrAg were subjected to the measurement, to prepare a standard curve. Based on the amount of luminescence from each specimen, the HBcrAg concentration (kU/mL) was calculated.

The results are shown in Table 7. Test Examples 31 and 32, in which HB124 or HB135 was added as an immobilized antibody, exhibited similar intensities of reaction with the specimen and the recombinant antigen of HBV genotype D.

Further, Test Examples 31 and 32, in which HB124 or HB135 was added as an immobilized antibody, exhibited about four times increases in the measured values for the specimens of HBV genotype E and genotype F in average compared to Test Example 30, in which these immobilized antibodies were not added.

It was shown that, by using the monoclonal antibody HB124 or HB135, the measurement sensitivities for genotypes E and F can be increased.

**[Table 7]**

| Specimen type | Genotype | HBcrAg (kU/mL) | | | Rate of increase | |
|---|---|---|---|---|---|---|
| | | Test Example 30 | Test Example 31 | Test Example 32 | | |
| | | - | HB124 | HB135 | HB124 | HB135 |
| A | A | 0.90 | 1.01 | 0.91 | 112% | 100% |
| C | C | 0.51 | 0.54 | 0.54 | 105% | 106% |
| D | D | 1.83 | 4.57 | 4.43 | 249% | 242% |
| rHBeAg(D) | D | 83.75 | 167.86 | 176.37 | 200% | 211% |
| E-1 | E | 0.53 | 1.83 | 1.79 | 344% | 335% |
| E-2 | E | 0.54 | 1.72 | 1.53 | 318% | 283% |
| E-3 | E | <0.12 | <0.12 | <0.12 | ND | ND |
| E-4 | E | <0.12 | <0.12 | <0.12 | ND | ND |
| E-5 | E | 0.13 | 0.50 | 0.43 | 401% | 344% |
| E-6 | E | <0.12 | 0.12 | <0.12 | increase | ND |
| E-7 | E | 0.51 | 2.00 | 1.95 | 389% | 379% |
| E-8 | E | 3.19 | 7.83 | 7.82 | 245% | 245% |
| E-9 | E | 0.33 | 0.95 | 0.87 | 286% | 264% |
| E-10 | E | <0.12 | 0.16 | 0.15 | Increase | Increase |
| E-11 | E | <0.12 | <0.12 | <0.12 | ND | ND |
| E-12 | E | 1.30 | 4.50 | 4.12 | 347% | 318% |
| E-13 | E | 13.06 | 46.29 | 44.42 | 354% | 340% |
| E-14 | E | 0.35 | 1.18 | 1.15 | 339% | 329% |
| E-15 | E | 0.34 | 1.04 | 0.92 | 306% | 272% |
| E-16 | E | <0.12 | <0.12 | <0.12 | ND | ND |
| E-17 | E | <0.12 | <0.12 | <0.12 | ND | ND |
| E-18 | E | 24410.62 | 29285.75 | 35846.40 | 120% | 147% |
| E-19 | E | 0.37 | 1.00 | 0.80 | 268% | 214% |
| E-20 | E | 4.78 | 16.44 | 15.82 | 344% | 331% |
| E-21 | E | 0.53 | 1.84 | 1.71 | 347% | 320% |
| E-22 | E | <0.12 | <0.12 | <0.12 | ND | ND |
| E-23 | E | 0.78 | 2.80 | 2.53 | 357% | 323% |
| E-24 | E | 3.17 | 7.92 | 6.95 | 250% | 219% |
| F-1 | F | 0.23 | 2.40 | 2.36 | 1027% | 1010% |
| F-2 | F | <0.12 | <0.12 | <0.12 | ND | ND |
| F-3 | F | <0.12 | 0.16 | 0.12 | Increase | Increase |
| F-4 | F | <0.12 | <0.12 | <0.12 | ND | ND |
| F-5 | F | <0.12 | <0.12 | <0.12 | ND | ND |
| F-6 | F | <0.12 | <0.12 | <0.12 | ND | ND |
| F-7 | F | 0.26 | 2.77 | 2.53 | 1087% | 993% |
| F-8 | F | <0.12 | <0.12 | <0.12 | ND | ND |
| F-9 | F | <0.12 | 0.30 | 0.26 | Increase | Increase |
| Average rate of increase | | | | | 354% | 333% |

| | | | | | | |
|---|---|---|---|---|---|---|
| ND: Impossible to calculate | | | | | | |

### EXAMPLE 7

### Pretreatment Including Alkaline Substance

To antigen solutions containing 0, 5, or 250 KU/mL of the recombinant HBcrAg, monoclonal antibodies HB44, HB124, HB61, HB114, and HB91 were added to 10 µg/ml each. The resulting mixtures were incubated at 37°C for 60 minutes to prepare competitive-antibody-positive model specimens. Antigen solutions free of the monoclonal antibodies were provided as competitive-antibody-negative model specimens. To 1.5-mL tubes, 40 µL of each of the competitive-antibody-positive model specimens and the competitive-antibody-negative model specimens was dispensed. After addition of 70 µL of 0 M to 0.5 M sodium hydroxide (NaOH) thereto, the resulting mixture was stirred, and the reaction was allowed to proceed at 37°C for 6.5 minutes. Neutralization was carried out by addition of 70 µL of a neutralization solution containing hydrochloric acid at a concentration equimolar to NaOH, 0.7 M urea, and 0.2% Triton X-100. Immediately thereafter, the resulting mixture was stirred to obtain a treated specimen.

Thirty microliters of the treated specimen was added to 50 µL of the particle dilution prepared in Example 3 containing 0.06% magnetic particles on which HB44, HB124, HB61, and HB114 were immobilized, and the reaction was allowed to proceed at 37°C for 8 minutes. The subsequent reactions were carried out by the method described in Example 3, to measure IIBcrAg.

As shown in Table 8, it could be confirmed that, by the presence of NaOH at not less than 0.127 M during the pretreatment, HBcrAg can be measured without being influenced by the competitive antibodies.

**[Table 8]**

| Model specimen | NaOH (M) during pretreatment: NaOH (M) : HBcrAg (KU/mL) | 0 | 0.0064 | 0.032 | 0.064 | 0.127 | 0.191 | 0.318 |
|---|---|---|---|---|---|---|---|---|
| | | 0 | 0.01 | 0.05 | 0.1 RLU | 0.2 | 0.3 | 0.5 |
| Competitive-antibody-negative model specimen | 0 | 1,281 | 1,397 | 1,311 | 1,403 | 1,486 | 1,702 | 1,566 |
| | 5 | 3,892 | 3,777 | 4,027 | 3,537 | 2,776 | 2,428 | 2,360 |
| | 250 | 131,138 | 131,964 | 139,402 | 123,891 | 74,208 | 52,721 | 30,708 |
| Competitive-antibody-positive model specimen | 0 | 1,416 | 1,321 | 1,391 | 1,413 | 1,395 | 1,618 | 1,714 |
| | 5 | 1,444 | 1,426 | 1,404 | 1,494 | 2,712 | 2,652 | 2,232 |
| | 250 | 4,709 | 4,874 | 4,917 | 4,763 | 70,141 | 51,180 | 31,215 |
| Antibody-positive model specimen / antibody-negative model specimen (%) | 0 | 111 | 95 | 106 | 101 | 94 | 95 | 109 |
| | 5 | 37 | 38 | 35 | 42 | 98 | 109 | 95 |
| | 250 | 4 | 4 | 4 | 4 | 95 | 97 | 102 |

### Treatment)

As surfactants to be combined with a pretreatment liquid containing an alkaline substance, nonionic surfactants (Triton X-100, Brij35, Tween 20, and Tween 80), zwitterionic surfactants (CHAPS, C12APS, C14APS, C16APS), and anionic surfactants (SDS, SDBS, NLS) were studied.

The pretreatment liquid was prepared as a mixture containing 0.2 M NaOH (concentration during the pretreatment, 0.127 M) and 0.16, 0.8, or 4% surfactant (concentration during the pretreatment, 0.1, 0.5, or 2.5%).

In 1.5-mL tubes, 40 µL of each of the competitive-antibody-positive model specimens (recombinant HBcrAg concentration, 250 KU/mL) and the competitive-antibody-negative model specimens (recombinant HBcrAg concentration, 250 KU/mL) prepared in Example 7; and 70 µL of the pretreatment liquid prepared by mixing NaOH and the surfactant; were mixed. After stirring the resulting mixture, the reaction was allowed to proceed at 37°C for 6.5 minutes. Neutralization was carried out by addition of 70 µL of a neutralization solution containing 0.2 M HCl. Immediately thereafter, the resulting mixture was stirred to obtain a treated specimen. To provide controls for the cases without alkaline treatment, 0.2 M NaCl solution was used instead of the above pretreatment liquid.

Measurement of HBcrAg in each treated specimen was carried out in the same manner as in Example 7.

By the presence of not less than 0.1% nonionic surfactant, zwitterionic surfactant, or anionic surfactant during the pretreatment, the precipitation due to the alkaline treatment could be reduced, and the influence of the competitive antibodies could be eliminated, resulting in increases in the sensitivity (Table 9).

The anionic surfactants showed larger increases in the sensitivity relative to the nonionic and zwitterionic surfactants.

**[Table 9]**

| | NaOH concentration (M) during pretreatment | Surfactant concentration (%) during pretreatment | | Comp etitive- anti body-negative model specimen (luminescence signal RLU) | Competitive- antibody-positive model specimen (luminescence signal RLU) | Competitive-anti body-negative model specimen relative to control (%) | Antibody-positive model specimen / antibody-negative model specimen (%) |
|---|---|---|---|---|---|---|---|
| Control | 0 | - | 0 | 18,683 | 3,244 | 100% | 17% |
| Nonionic surfactant | 0.127 | Tween 20 | 0.1 | 44,799 | 36,503 | 240% | 81% |
| | | | 0.5 | 61,157 | 51,337 | 327% | 84% |
| | | | 2.5 | 26,575 | 33,590 | 142% | 126% |
| | | Triton X-100 | 0.1 | 57,744 | 52,368 | 309% | 91% |
| | | | 0.5 | 91,929 | 82,303 | 492% | 90% |
| | | | 2.5 | 113,990 | 97,355 | 610% | 85% |
| | | Brij35 | 0. 1 | 54,398 | 46,070 | 291% | 85% |
| | | | 0.5 | 153,192 | 155,864 | 820% | 102% |
| | | | 2.5 | 117,830 | 121,105 | 631% | 103% |
| | | Tween 80 | 0.1 | 60,297 | 53,370 | 323% | 89% |
| | | | 0.5 | 104,891 | 66,683 | 561% | 64% |
| | | | 2.5 | 183,649 | 117,258 | 983% | 64% |
| Zwitterionic surfactant | 0.127 | CHAPS | 0.1 | 31,292 | 27,815 | 167% | 89% |
| | | | 0.5 | 38,282 | 45,056 | 205% | 118% |
| | | | 2.5 | 39,201 | 41,155 | 210% | 105% |
| | | C12APS | 0.1 | 39,175 | 61,507 | 210% | 157% |
| | | | 0.5 | 89,259 | 122,530 | 478% | 137% |
| | | | 2.5 | 128,918 | 133,713 | 690% | 104% |
| | | C14APS | 0.1 | 73,879 | 94,203 | 395% | 128% |
| | | | 0.5 | 133,424 | 143,293 | 714% | 107% |
| | | | 2.5 | 154,272 | 143,421 | 826% | 93% |
| | | C16APS | 0.1 | 82,813 | 87,113 | 443% | 105% |
| | | | 0.5 | 169,484 | 167,820 | 907% | 99% |
| | | | 2.5 | 98,203 | 93,705 | 526% | 95% |
| Anionic surfactant | 0.127 | SDS | 0.1 | 103,718 | 81,976 | 555% | 79% |
| | | | 0,5 | 160,219 | 118,036 | 858% | 74% |
| | | | 2.5 | 258,650 | 267,299 | 1384% | 103% |
| | | | 4 | 256,126 | 255,915 | 1533% | 100% |
| | | | 5 | 248,475 | 241,434 | 1488% | 97% |
| | | SDBS | 0.1 | 91,940 | 73,524 | 492% | 80% |
| | | | 0.5 | 144,734 | 98,114 | 775% | 68% |
| | | | 2.5 | 216,134 | 206,387 | 1157% | 95% |
| | | NLS | 0.1 | 85,782 | 75,072 | 459% | 88% |
| | | | 0.5 | 132,041 | 107,507 | 707% | 81% |
| | | | 2.5 | 262,628 | 227,581 | 1406% | 87% |
| | | | 4 | 268,971 | 253,247 | 1610% | 94% |
| | | | 5 | 268,041 | 252,405 | 1605% | 94% |

### EXAMPLE 9

### Effect of Addition of Surfactant and Reducing Agent to Specimen Pretreatment Liquid (Alkaline Treatment)

To two kinds of purchased HBcrAg-positive specimens with known antigen concentrations (Specimen A × 10⁴ dilution, 4.46 LogU/mL (p22crAg-dominant specimen) and Specimen B × 10⁴ dilution, 4.74 LogU/mL (HBeAg-dominant specimen)), monoclonal antibodies HB44, HB124, HB61, HB114, and HB91 were added to 10 µg/mL each. The resulting mixtures were incubated at 37°C for 60 minutes to prepare competitive-antibody-positive specimens. Specimens free of the monoclonal antibodies were provided as competitive-antibody-negative specimens.

To the pretreatment liquid of 0.2 M (0.127 M during the pretreatment) NaOH and 4.71% (3% during the pretreatment) SDS, DEAET or TCEP was added as a reducing agent such that its concentration became 1, 3, 6, or 10 mM during the pretreatment, to prepare a pretreatment liquid. To 40 µL of each of the competitive-antibody-positive specimens and the competitive-antibody-negative specimens, 70 µL of the pretreatment liquid, prepared by mixing NaOH, SDS, and each concentration of DEAET or TCEP, was added. After stirring the resulting mixture, the reaction was allowed to proceed at 37°C for 6.5 minutes. Neutralization was carried out by addition of 70 µL of a neutralization solution containing 0.2 M HCl, and the resulting mixture was stirred immediately thereafter. To provide controls, the same treatment was carried out with 0 mM reducing agent.

Measurement of HBcrAg in each treated specimen was carried out in the same manner as in Example 7.

By the addition of the reducing agents, increases in the sensitivity due to the pretreatment were found. The addition was found to be especially effective for Specimen A (p22cr-dominant specimen) (Table 10).

**[Table 10]**

| | NaOH concentration (M) during pretreatment | SDS concentration (%) during pretreatment | Reducing agent concentration during pretreatment (mM) | | Competitive-antibody-negative specimen (RLU) | Competitive-anti body-positive specimen (RLU) | Competitive-antibody-negative specimen relative to control (%) | Antibody-positive specimen / antibody -negative specimen (%) |
|---|---|---|---|---|---|---|---|---|
| Specimen A (4.46 LU/ml) | 0.127 | 3 | - | 0 | 43,246 | 43,164 | 100% | 100% |
| | 0.127 | 3 | DEAET | 1 | 49,366 | 50,756 | 114% | 103% |
| | | | | 3 | 55,838 | 62,646 | 129% | 112% |
| | | | | 6 | 55,446 | 51,641 | 128% | 93% |
| | | | | 10 | 49,620 | 53,879 | 115% | 109% |
| | 0.127 | 3 | TCEP | 1 | 45,201 | 48,774 | 105% | 108% |
| | | | | 3 | 53,050 | 53,474 | 123% | 101% |
| | | | | 6 | 67,812 | 70,529 | 157% | 104% |
| | | | | 10 | 68,061 | 79,628 | 157% | 117% |
| Specimen B (4.74 LU/ml) | 0.127 | 3 | - | 0 | 59,238 | 58,010 | 100% | 98% |
| | 0.127 | 3 | DEAET | 1 | 58,983 | 59,073 | 100% | 100% |
| | | | | 3 | 62,332 | 61,635 | 105% | 99% |
| | | | | 6 | 57,565 | 60,195 | 97% | 105% |
| | | | | 10 | 49,892 | 52,169 | 84% | 105% |
| | 0.127 | 3 | TCEP | 1 | 58,495 | 59,146 | 99% | 101% |
| | | | | 3 | 58,793 | 59,833 | 99% | 102% |
| | | | | 6 | 69,117 | 69,236 | 117% | 100% |
| | | | | 10 | 69,152 | 70,752 | 117% | 102% |

## Claims

1. A method of immunoassay of hepatitis B virus core-related antigen, the method comprising using, as an antibody to be used for the immunoassay, a monoclonal antibody that specifically binds to at least one kind of core-related antigen of hepatitis B virus genotype D, or an antigen-binding fragment thereof, wherein an epitope of the monoclonal antibody or the antigen-binding fragment thereof is a region included in the amino acid sequence from position 31 to 48 of SEQ ID NO:3.

2. The method according to claim 1, wherein a monoclonal antibody that specifically binds to core-related antigen of hepatitis B virus genotype C, or an antigen-binding fragment thereof, is also used as an antibody to be used for the immunoassay.

3. The method according to claim 1 or 2, wherein the immunoassay is a sandwich method including a first antibody and a second antibody that specifically bind to hepatitis B virus core-related antigen,
the first antibody being a capture antibody bound to a solid phase, the second antibody being a detection antibody bound to a labeling substance,
wherein at least one of the first antibody and the second antibody is the monoclonal antibody that specifically binds to the core-related antigen of hepatitis B virus genotype D.

4. The method according to claim 3, wherein a solution containing the second antibody comprises a water-soluble polymer.

5. The method according to any one of claims 1 to 4, comprising pretreating a test sample with a pretreatment liquid containing at least one selected from the group consisting of a surfactant, an acidifier, and an alkaline substance.

6. The method according to claim 5, wherein the pretreatment liquid further contains a reducing agent.

7. The method according to any one of claims 1 to 6, wherein the hepatitis B virus core-related antigen to be assayed by the immunoassay is a hepatitis B virus core-related antigen of genotype D.

8. The method according to any one of claims 1 to 6, wherein the hepatitis B virus core-related antigen to be assayed by the immunoassay is a hepatitis B virus core-related antigen of genotype E or F.

9. A kit for immunoassay of hepatitis B virus core-related antigen, the kit comprising, as an antibody to be used for the immunoassay, a monoclonal antibody capable of binding reaction with a core-related antigen of hepatitis B virus genotype D, or an antigen-binding fragment thereof, wherein an epitope of the monoclonal antibody or the antigen-binding fragment thereof is a region included in the amino acid sequence from position 31 to 48 of SEQ ID NO:3.

10. The kit according to claim 9, further comprising a monoclonal antibody that specifically binds to a core-related antigen of hepatitis B virus genotype C, or an antigen-binding fragment thereof, as an antibody to be used for the immunoassay.


SEQUENCE LISTING

<110>  FUJIREBIO INC.

<120>  Immunoassay of hepatitis B virus core-related antigen and kit
       therefor

<130>  PF000675-PCT

<160>  6

<170>  PatentIn version 3.5

<210>  1
<211>  183
<212>  PRT
<213>  hepatitis B virus

<400>  1

Met Asp Ile Asp Pro Tyr Lys Glu Phe Gly Ala Ser Val Glu Leu Leu
1               5                   10                  15

Ser Phe Leu Pro Ser Asp Phe Phe Pro Ser Ile Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Asn Leu Ala Thr Trp Val Gly Ser Asn Leu Glu Asp Pro Ala
65                  70                  75                  80

Ser Arg Glu Leu Val Val Ser Tyr Val Asn Val Asn Met Gly Leu Lys
                85                  90                  95

Ile Arg Gln Leu Leu Trp Phe His Ile Ser Cys Leu Thr Phe Gly Arg
            100                 105                 110

Glu Thr Val Leu Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
                180

<210>  2
<211>  159
<212>  PRT
<213>  Hepatitis B virus

<400>  2

Ser Lys Leu Cys Leu Gly Trp Leu Trp Gly Met Asp Ile Asp Pro Tyr
1               5                   10                  15

Lys Glu Phe Gly Ala Ser Val Glu Leu Leu Ser Phe Leu Pro Ser Asp
            20                  25                  30

Phe Phe Pro Ser Ile Arg Asp Leu Leu Asp Thr Ala Ser Ala Leu Tyr
        35                  40                  45

Arg Glu Ala Leu Glu Ser Pro Glu His Cys Ser Pro His His Thr Ala
    50                  55                  60

Leu Arg Gln Ala Ile Leu Cys Trp Gly Glu Leu Met Asn Leu Ala Thr
65                  70                  75                  80

Trp Val Gly Ser Asn Leu Glu Asp Pro Ala Ser Arg Glu Leu Val Val
                85                  90                  95

Ser Tyr Val Asn Val Asn Met Gly Leu Lys Ile Arg Gln Leu Leu Trp
            100                 105                 110

Phe His Ile Ser Cys Leu Thr Phe Gly Arg Glu Thr Val Leu Glu Tyr
        115                 120                 125

Leu Val Ser Phe Gly Val Trp Ile Arg Thr Pro Pro Ala Tyr Arg Pro
    130                 135                 140

Pro Asn Ala Pro Ile Leu Ser Thr Leu Pro Glu Thr Thr Val Val
145                 150                 155

<210>  3
<211>  149
<212>  PRT
<213>  hepatitis B virus

<400>  3

Met Asp Ile Asp Pro Tyr Lys Glu Phe Gly Ala Ser Val Glu Leu Leu
1               5                   10                  15

Ser Phe Leu Pro Val Asp Phe Phe Pro Ser Val Arg Asp Leu Leu Asp
            20                  25                  30

Thr Ala Ser Ala Leu Tyr Arg Asp Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Ala Asn Leu Glu Asp Pro Ala
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Thr Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Gln Asn Ala Pro Ile Leu Ser Thr Leu Pro
    130                 135                 140

Glu Thr Thr Val Val
145

<210>  4
<211>  18
<212>  PRT
<213>  hepatitis B virus

<400>  4

Leu Asp Thr Ala Ser Ala Leu Tyr Arg Asp Ala Leu Glu Ser Pro Glu
1               5                   10                  15

His Cys

<210>  5
<211>  27
<212>  DNA
<213>  artificial

<220>
<223>  forward PCR primer

<400>  5
gaattcatgg acattgaccc gtataaa                                          27

<210>  6
<211>  26
<212>  DNA
<213>  artificial

<220>
<223>  reverse PCR primer

<400>  6
ggatcctaac attgagattc ccgaga                                          26